Europäisches Patentamt

European Patent Office

Office européen des brevets

(19)

(11) Numéro de publication : **0 481 987 B1**

(12) **FASCICULE DE BREVET EUROPEEN**

(45) Date de publication du fascicule du brevet :
29.11.95 Bulletin 95/48

(51) Int. Cl.⁶ : **H04N 5/32**

(21) Numéro de dépôt : **89909003.9**

(22) Date de dépôt : **13.07.89**

(86) Numéro de dépôt international :
**PCT/FR89/00375**

(87) Numéro de publication internationale :
**WO 91/01071 24.01.91 Gazette 91/03**

(54) PROCEDE DE CORRECTION DE LA DISTORSION D'IMAGES RADIOLOGIQUES.

(43) Date de publication de la demande :
29.04.92 Bulletin 92/18

(45) Mention de la délivrance du brevet :
29.11.95 Bulletin 95/48

(84) Etats contractants désignés :
DE GB NL

(56) Documents cités :
EP-A- 0 021 366
EP-A- 0 293 293
DE-A- 3 419 043
US-A- 4 736 399
Proceedings of 1986 IEEE International Conference on Robotics and Automation, 7-10 April 1986, San Francisco (CA, US), vol. 1, IEEE, N. Yokobori et al.: "Sub-pixel geometric correction of pictures by calibration and decalibration", pages 448-453

(73) Titulaire : **GENERAL ELECTRIC CGR S.A.**
**100, rue Camille-Desmoulins**
**F-92130 Issy les Moulineaux (FR)**

(72) Inventeur : **LECLERC, Vincent**
**49, rue d'Alésia**
**F-75014 Paris (FR)**
Inventeur : **PICARD, Catherine**
**51, rue Pergolèse**
**F-75116 Paris (FR)**
Inventeur : **LAVAYSSIERE, Blandine**
**Résidence Sylvabelle**
**1, route de Thierry**
**F-92410 Ville-d'Avray (FR)**

(74) Mandataire : **Schmit, Christian Norbert Marie et al**
**Cabinet Ballot-Schmit**
**7, rue Le Sueur**
**F-75116 Paris (FR)**

## Description

La présente invention a pour objet un procédé de correction de la distorsion d'images radiologiques acquises avec un tube intensificateur de luminance. Elle s'applique plus particulièrement dans le domaine médical. Elle peut être mise en oeuvre soit en radioscopie directe, soit en radiologie avec traitement numérisé du signal représentatif de l'image. Elle concerne plus particulièrement des tomodensitomètres de générations futures dont l'organe de détection sera un tel tube intensificateur de luminance. Elle a pour objet de résoudre les problèmes de morphométrie que présente l'utilisation de tels tubes.

Un tube intensificateur d'images radiologiques est destiné à recevoir un rayonnement X de faible puissance et à transformer ce rayonnement X en un rayonnement lumineux plus puissant, plus facilement détectable par un moyen de visualisation, en particulier par une caméra. La raison de la faiblesse du rayonnement X reçu est à rechercher dans le soucis de protéger, en particulier dans le domaine médical, les patients soumis à des examens avec de tels rayonnements. Ceci se présente spécialement lorsque ces examens sont longs, comme c'est le cas des traitements de tomodensitométrie ou des traitements avec numérisation des informations d'images.

Un tube intensificateur d'images comporte essentiellement une dalle de conversion pour convertir un rayonnement X reçu en un rayonnement lumineux susceptible d'attaquer une photocathode placée en vis à vis de cette dalle. La transformation rayonnement X-rayonnement lumineux est obtenue d'une manière connue en munissant la dalle de cristaux d'iodure de Césium. Sous l'effet de l'illumination X, des photo-électrons sont arrachés de la photo-cathode et se déplacent en direction d'un écran. Ce déplacement vers l'écran est soumis aux effets d'une optique électronique. Cette optique électronique tend à ce que les impacts des photo-électrons sur l'écran correspondent aux lieux de la photo-cathode d'où ils ont été émis.

L'écran est lui-même d'un type particulier : il réémet une image lumineuse représentative de l'image électronique transportée par les électrons, elle-même représentative de l'image de rayons X. Cette image lumineuse peut alors être détectée par un moyen de visualisation quelconque, en particulier une caméra classique, de manière à être visualisée sur un dispositif de visualisation, en particulier un dispositif de type moniteur de télévision.

Une telle chaîne de visualisation présente un inconvénient important : l'image révélée est une image distordue géométriquement par rapport à l'image de rayons X qui en est l'origine. Cette distorsion se produit essentiellement entre la photo-cathode, excitée par les photons émanant de la dalle de conversion, et l'écran qui reçoit le rayonnement électronique émis par cette photo-cathode. En effet, pendant leur transit, les photo-électrons sont soumis à des effets perturbateurs, notamment à des effets magnétiques, dus au champ magnétique terrestre. Si tous les photo-électrons pendant ce transit étaient affectés par un même type de perturbation, il suffirait de corriger à un endroit quelconque de la chaîne d'images l'action de ces perturbations pour ne pas en être gêné. Malheureusement la sensibilité de ces photo-électrons aux perturbations est forte. Et l'inhomogénéité du champ magnétique sur leurs lieux de passage est alors telle qu'il en résulte une distorsion dans l'image électronique projetée sur l'écran.

Pour rendre plus concrets les effets d'une telle distorsion, on peut dire que l'image d'une droite interposée entre un tube à rayons X et un tel intensificateur d'images sera une droite dans l'image des rayons X qui excitent la dalle, sera une droite dans l'image photonique qui attaque la photo-cathode, sera une droite dans l'image électronique qui quitte cette photo-cathode, mais ne sera plus une droite dans l'image électronique qui vient s'afficher sur l'écran. Par conséquent elle ne pourra plus être une droite dans l'image lumineuse produite par cet écran. Le dispositif de visualisation que l'on place en aval révèle alors en quelque sorte le résultat de la distorsion due à l'inhomogénéité du champ magnétique terrestre dans l'espace traversé par l'image électronique.

Jusqu'à présent, ce type d'inconvénient avait pu être négligé du fait que les images que l'on cherchait à produire étaient essentiellement qualitatives et qu'on se préoccupait peu de leur contenu quantitatif : de la justesse du dessin des contours des objets révélés. Cependant, actuellement, avec le développement des techniques on cherche de plus en plus à utiliser de telles images d'une manière quantitative. Par exemple on peut vouloir réaliser des prothèses à partir des images obtenues, et il est alors intolérable de disposer d'images faussées. Par ailleurs, dans le contrôle industriel, ce type de défaut entraîne l'impossibilité d'utiliser facilement de tels intensificateurs d'images en métrologie.

Parmi les déformations ou distorsions de l'image, on met en évidence une déformation dite "en coussin" provenant de la géométrie de la calotte sphérique de la face d'entrée du tube, de la face supérieure de la dalle. On met aussi en évidence une déformation dite en "S" provenant de la déviation des trajectoires électroniques par les champs magnétiques, en particulier le champ magnétique terrestre. La distorsion présente donc une composante permanente, liée à un tube donné, et une composante variable, liée à la position même du tube dans le champ magnétique terrestre.

Différents procédés ont été envisagés pour réduire les effets de cette dernière distorsion. Dans une première approche, par des développements technologiques on a tenté de réduire les effets de distorsion, notamment les effets des champs magnétiques perturbateurs. A cette fin, on a muni les tubes intensificateurs d'images de blindages magnétiques (canalisateurs du champ magnétique) enveloppant le tube. Mais cette enveloppe ne peut pas recouvrir la dalle de conversion de telle sorte que les effets magnétiques perturbateurs s'exercent quand même à proximité de cette dalle, là où ils sont en définitive les plus efficients du fait que les photo-électrons arrachés de la photo-cathode sont, à proximité de cette dalle, animés de vitesses très faibles.

Pour compléter ce dispositif, il a par ailleurs été imaginé de disposer à proximité de la face supérieure du tube, une bobine de production d'un champ magnétique compensateur. Dans une demande de brevet français n° 88 04071 déposée le 29 mars 1988, il a même été envisagé d'asservir le courant qui parcourait cette bobine à une mesure du champ magnétique à compenser. Malgré tout l'intérêt que présente cette méthode elle ne donne que des résultats imparfaits. La précision de la correction de distorsion est insuffisante eu égard aux applications envisagées car elle non plus ne permet pas d'éliminer la distorsion "en coussin"..

Une autre méthode de correction des distorsions a été envisagée. Elle concerne une approche paramétrique. Selon cette approche, on modélise les déformations en se basant sur la connaissance des caractéristiques géométriques et électro-optiques du système. Le succès de cette méthode est conditionné par la précision de la connaissance du système que l'on désire modelise. En tant qu'approche analytique elle nécessite pour pouvoir être calculable, d'importantes simplifications du modèle. Ces simplifications sont telles que cette méthode ne peut plus prendre en compte finalement tous les phénomènes, en particulier ceux plus complexes résultant de la déformation en "S".

L'invention a pour objet de remédier à ces inconvénients en proposant une méthode nouvelle dans laquelle on procède à l'acquisition d'images d'une mire calibrée et dans laquelle on mesure la distorsion de l'image de cette mire par rapport à son allure théorique attendue. On effectue bien entendu cette mesure pour toutes les positions utiles dans l'espace de l'ensemble tube intensificateur d'images-tube à rayons X. Ultérieurement on corrige les images radiologiques acquises en fonction des évaluations de cette distorsion. Cette méthode pragmatique et non paramétrique permet de prendre en compte tous les phénomènes physiques intervenant dans la distorsion par opposition aux méthodes connues où d'une manière (technologie grossière) ou d'une autre (modélisation approchée) on ne prenait pas en compte la totalité des distorsions. Un tel procédé est par exemple décrit dans le brevet US-A-4 736 399 en ce qui concerne les écrans intensificateurs d'images radiologiques ou dans la demande de brevet EP-A-0 021 366 en ce qui concerne les gamma caméras. Néanmoins la précision obtenue dans ces deux cas est insuffisante. L'invention procure une précision bien meilleure, même, que la taille d'un pixel.

L'invention a donc pour objet un procédé de correction de la distorsion d'images radiologiques acquises avec un tube intensificateur de luminance, ces images comportant une collection d'adresses de points d'image en relation avec des niveaux de gris attribués à ces points, dans lequel

on acquiert l'image réelle d'une mire placée sur la face d'entrée de ce tube,

on évalue la distorsion de cette image de la mire par rapport à son allure théorique attendue,

et on corrige des images radiologiques normales en fonction de cette évaluation,
caractérisé en ce que pour acquérir l'image réelle de la mire

- on réalise une mire amovible par photogravure (79) de montants en une couche métallique,
- on recherche automatiquement dans l'image réelle les montants de la mire, et
- on calcule dans l'image réelle de la mire la position du barycentre de l'illumination X d'un montant de cette mire de façon à obtenir une précision en fraction de pixel sur les coordonnées des montants de la mire.

Dans un perfectionnement l'acquisition de l'image réelle d'une mire, compte tenu de la multiplicité des positions possibles dans l'espace de l'ensemble tube intensificateur - tube à rayons X, est automatique. Dans l'invention, l'acquisition est faite en utilisant une mire particulière dont la structure est telle qu'elle n'introduit pas elle-même des imprécisions dans les calculs de correction qu'on cherche à en déduire.

L'invention est utilisée en particulier dans les systèmes d'angiographie numérique où il est souhaitable d'estimer des déformations géométriques afin de corriger des mesures de distance, de surfaces ou de volumes, réalisées sur les organes à partir des images, mais où il est également intéressant de restaurer effectivement les images. Cette restauration a un intérêt en artériographie des membres inférieurs dans le cas où on veut reconstruire une jambe à partir de plusieurs images juxtaposées par exemple.

En tomodensitométrie généralisée, dans le cas de reconstruction à trois dimensions, dite 3D, à partir de projections à deux dimensions dites 2D, la correction des distorsions est indispensable et nécessite une précision importante. La précision intervient à deux moments : lors de la calibration du système d'acquisition, et lors de la restauration des images proprement dite, préalablement à toute reconstruction 3D. On pourra montrer qu'on peut obtenir avec l'invention les précisons requises : c'est-à-dire des précisions de 1/10ème de point d'image pour la calibration du système d'acquisition, et de 1/2 point d'image pour la reconstruction 3D à partir des projections 2D. En outre, on peut se satisfaire des corrections de l'ordre du pixel pour les mesures géométriques sur l'image.

L'invention sera mieux comprise à la lecture de la description qui suit et à l'examen des figures qui l'accompagnent. Celles-ci ne sont données qu'à titre indicatif et nullement limitatif de l'invention. Les figures montrent :

- figure 1 : la représentation schématique d'une chaîne radiologique pour mettre en oeuvre le procédé de l'invention ;
- figure 2 : la représentation d'une grille type utilisable comme mire ;
- figure 3 : la représentation graphique d'opérations de morphologie mathématique permettant la détection automatique de l'image de la mire ;
- figure 4 : la représentation schématique des corrections de distorsion à appliquer pour restaurer géométriquement les images ;
- figure 5 : la succession des opérations préférées mises en oeuvre dans le procédé de l'invention ;
- figure 6 : Les effets d'un traitement particulier des images pour stationnariser le fond et pour éliminer des bruits de fond ;
- figures 7a et 7b : La mise en évidence de caractéristiques technologiques conduisant à la détermination de la meilleure mire possible.

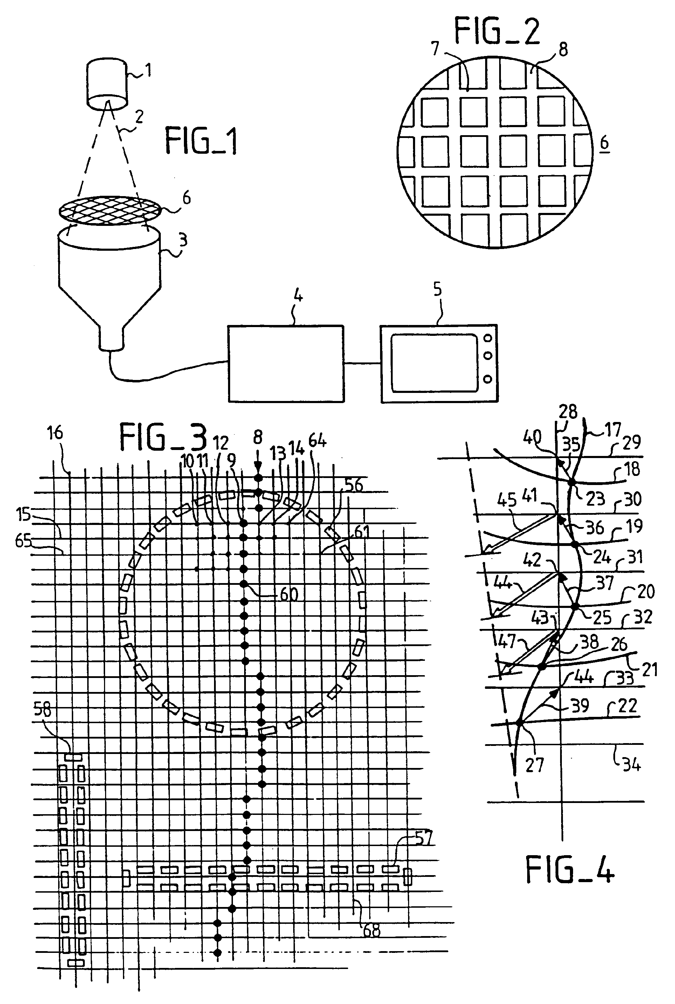

La figure 1 représente schématiquement une chaîne d'imagerie utilisable pour mettre en oeuvre le procédé de l'invention. Dans cette chaîne, un tube 1 à rayons X émet un rayonnement X 2 à direction d'un tube intensificateur d'images 3. Le tube intensificateur d'images 3 est relié à un dispositif de traitement d'image 4, lui-même relié à un dispositif 5 de visualisation des images traitées. Le relevé des distorsions subies par les images pour une position donnée de l'ensemble tube 1 à rayons X - tube 3 intensificateur d'images est donné par l'interposition, préalablement à toute mesure d'utilisation normale, d'une mire 6 entre ce tube 1 et l'intensificateur 3. De préférence la mire 6 a sensiblement les mêmes dimensions que la face d'entrée de l'intensificateur 3 et elle est placée contre cette face. En pratique la mire 6 comporte un quadrillage (figure 2) de montants horizontaux 7 et verticaux 8. Dans un exemple préféré la mire a une forme extérieure ronde, de diamètre environ 30 cm, et comporte des montants espacés les uns les autres d'environ 1cm et dont la largeur est de l'ordre de 1 mm.

Lors d'une opération de mesure de distorsion on enregistre dans la mémoire d'image du dispositif 4, et on peut le visualiser sur l'écran du moniteur 5, une image ressemblant à celle de la figure 3. Cette image comporte une collection d'adresses de points mémoires, correspondant aux points d'images (ou pixels) de l'image visualisée, associés à des informations représentatives de niveaux de gris. Ces niveaux de gris sont affichables par le spot du moniteur, sur l'écran de ce moniteur, et correspondent en définitive à la luminosité attribuée à ces points. Les montants de la mire, ici par exemple un montant 8, sont distordus (ici d'une manière exagérée).

On remarque, en plus du caractère géométrique distordu de l'image du montant 8, que ce montant est représenté par des pixels dont le niveau de gris est élevé, par exemple le pixel 9, et par des pixels dont le niveau de gris est faible, par exemple les pixels 10 à 14. Ces derniers sont représentés par des petits points par opposition aux gros points montrant sensiblement le centre du montant 8 à l'endroit d'un profil 15 coupant perpendiculairement le montant 8. De part et d'autre de l'endroit où se trouvent les pixels 10 et 14 dont le niveau de gris est faible, la mémoire d'images comporte des éléments d'images dont le niveau de gris est quasi nul (au bruit près). Ceci est schématisé par l'absence de points à l'intersection de traits 16 et 15 symbolisant des abscisses et des ordonnées (des adresses) de points d'image.

La figure 4 montre en substance le principe général de l'invention. Comme on le verra ultérieurement on va pouvoir calculer d'une manière précise les coordonnées, les adresses, de tous les points d'images représentatifs des centres des montants respectivement 17 à 22 verticaux et horizontaux de la mire. On montrera également qu'on peut calculer d'une manière automatique les adresses des points d'intersection 23 à 27 de tous les montants entre eux. En supposant que la mire occupe une position connue à l'avance, représentée par exemple par les montants 28 à 34, il devient possible de calculer les déplacements δ respectivement 35 à 39 à affecter aux coordonnées des points des images réelles qui se trouveraient respectivement en 23 à 27, pour les amener en des lieux 40 à 44 où la distorsion géométrique pourrait être considérée comme ayant été supprimée.

Pour des points des images réelles situés en position intermédiaire entre des points tels que 23 à 27, une interpolation bi-linéaire de correction est proposée de manière à leur attribuer un déplacement δ qui tienne compte de leur entourage immédiat.

On peut objecter que la position de la mire 6 sur l'intensificateur 3 n'est pas connue et qu'en définitive, on ne connaît pas exactement les positions des images théoriques 28 à 34 de ces montants ni de celles de leurs intersections. Mais on peut remarquer que la grille elle même est connue. Si on fait alors l'hypothèse supplémentaire que la mire 6 est munie de montants répartis d'une manière particulière, par exemple régulièrement, on peut admettre que les déplacements δ 35 à 39 sont connus, d'une part à un décalage entier près du pas de la grille près, et d'autre part à un changement d'axe près. Ce changement d'axe prend en compte l'orientation réelle des axes de la mire par rapport aux directions qu'on leur attribue arbitrairement dans la mémoire image. On remarque alors que ces deux approximations ne sont pas gênantes puisqu'elles affectent tous les points d'images soit de la même façon, soit d'une manière cohérente. En effet, la même translation d'un nombre entier de pas de la grille et les effets d'un même changement d'axes seraient à appliquer à toutes les intersections théoriques 40 à 44.

On a représenté par exemple en tirets sur la figure 4 la position non distordue et réelle que devraient occuper des axes théoriques 28 à 29 représentant l'image virtuelle de la mire. L'ensemble des translations et des changements d'axes est représenté par les doubles flèches 45 à 47. On remarque que ces doubles flèches n'ont pas nécessairement toutes la même longueur. Ceci signifie en définitive que l'on est pas obligé d'avoir placé la mire 6 d'une manière telle que ses différents montants se retrouvent ultérieurement rigoureusement parallèles aux lignes et aux colonnes d'images de la mémoire d'image ou de l'image visualisée.

Il est par contre important de remarquer que si l'on prend la précaution d'acquérir toutes les images de correction, pour toutes les positions de l'ensemble tube 1 à rayons X - intensificateur 3, avec une mire 6 maintenue fixe sur le tube 3 constante au cours des diverses acquisitions, les effets de ces translations et (changements d'axes) 45 à 47 seront les mêmes dans toutes les images. En définitive la manière dont la mire 6 aura été placée sur l'intensificateur 3 au moment de ces acquisitions de corrections n'aura pas d'influence.

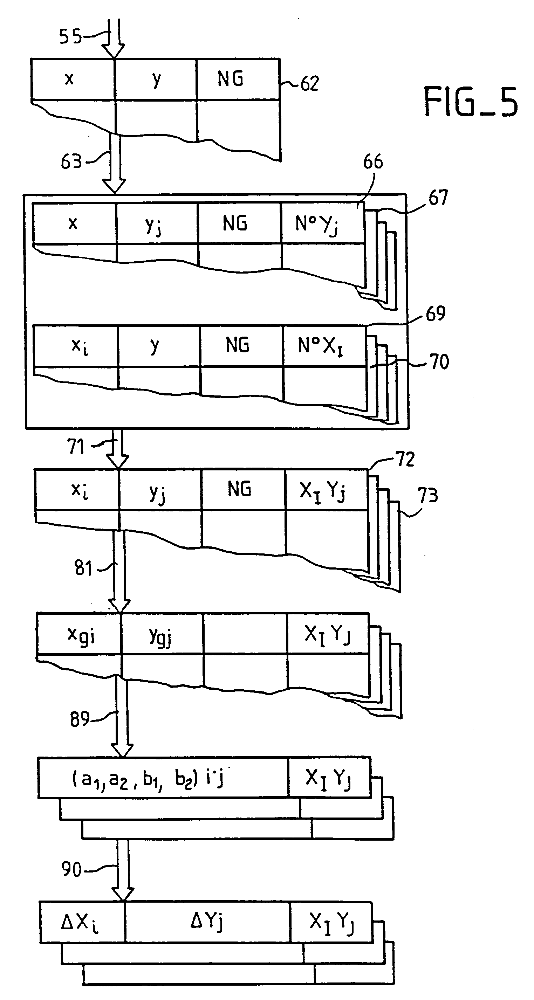

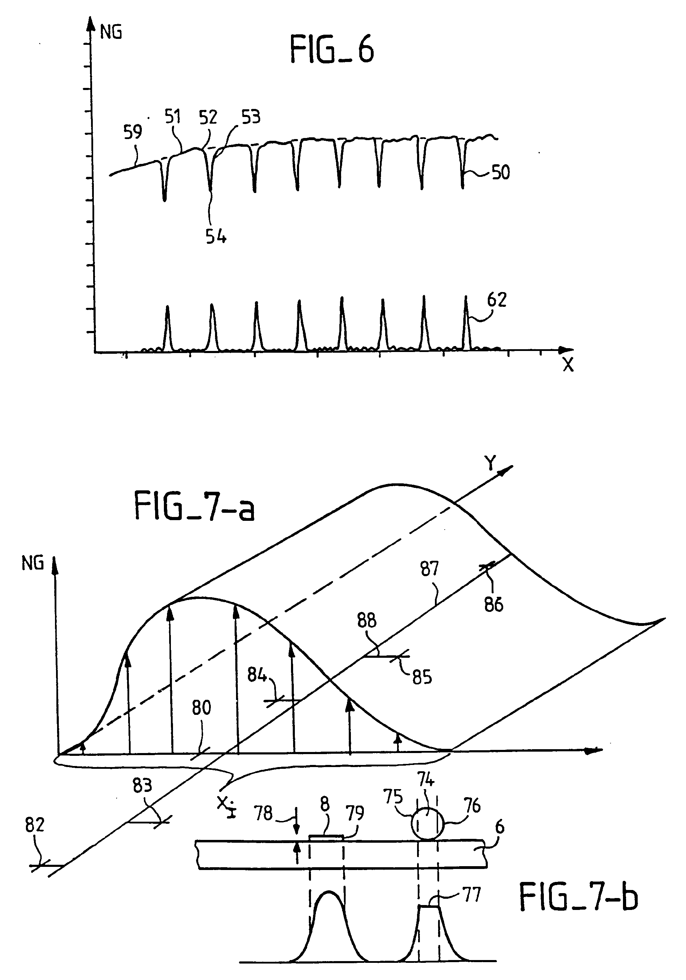

La figure 5 représente schématiquement l'ensemble des étapes du procédé de l'invention. Celui-ci sera expliqué en référence aux figures 6 et 3 qui correspondent à la des montants dans l'image numérique contenue dans la mémoire image. La figure 6 montre un tracé 50 obtenu par exploration de la mémoire image, par exemple le long d'un profil tel que le profil 15. Le long de ce profil on rencontre des adresses (c'est-à-dire des points) dont l'ordonnée est constante mais dont l'abscisse varie d'une unité d'un pixel à l'autre, d'un point image à l'autre. Dans des premières régions 51 le niveau de gris est élevé. En effet dans ces régions 51, correspondant à des espaces inter-montants, le rayonnement X a beaucoup excité l'intensificateur 3. Le signal électrique détecté correspondant a été élevé. Par contre, de part et d'autre, 52 et 53, du milieu 54 du montant, par exemple du montant 8, on trouve des régions où le niveau de gris baisse. Ces régions correspondent aux lieux des pixels 10 à 14. Le milieu 54 du montant correspond lui, schématiquement, au pixel 9.

On remarque que le signal 50 présente une variation de sa composante continue au fur et à mesure de l'exploration du profil selon l'axe des abscisses. Cette évolution est liée à la présence d'un fond d'image non stationnaire. Ce fond en définitive est gênant. En effet ce fond non stationnaire empêche la mise en oeuvre d'un seuil de détection simple pour repérer automatiquement la position des montants le long des profils. Dans l'invention, lorsque cela est nécessaire, au cours d'une opération cotée 55 sur la figure 5, on stationnarise l'image en appliquant à cette image de préférence une transformation morphologique dite "chapeau haut de forme".

Une telle transformation morphologique utilise des opérations de type ouverture et de fermeture qui sont des transformations propres à la théorie de la morphologie mathématique. On rappelle qu'une opération de fermeture est constituée d'une dilatation suivie d'une érosion. A l'opposé, une ouverture est constituée d'une érosion, suivie d'une dilatation. On rappelle qu'une érosion d'une image consiste à créer une autre image après une exploration en érosion de l'image de base. On explore l'image de base au moyen d'une fenêtre de dimensions et de forme géométrique données et possédant un centre donné. Par exemple dans l'invention, on utilisera trois types préférés de fenêtres (figure 3) : premièrement une fenêtre circulaire 56 constituée par un cercle de rayon 5 pixels, et deuxièmement des fenêtres de segment, respectivement 57 et 58. La longueur de ces segments est de 9 pixels et leur largeur n'est que de 1 pixel. On sait donner à ces segments 57 et 58 ainsi qu'au cercle 56 un centre. Ce centre peut être un point quelconque de la fenêtre. De préférence il s'agit d'un pixel situé au centre géométrique.

Lorsqu'on explore l'image de base avec une fenêtre, on évalue le niveau de gris de tous les pixels de cette image situés à l'aplomb de cette fenêtre. Dans une position d'exploration donnée, les niveaux de gris des pixels à l'aplomb peuvent être compris entre $\alpha$ qui est une valeur minimum et A qui est une valeur maximum. Dans une opération d'érosion, on décide d'attribuer au point de l'image à créer, qui a les mêmes coordonnées que le pixel au centre de la fenêtre dans l'image de base, un niveau de gris égal à $\alpha$. Dans une opération de dilatation on attribue au pixel de l'image à créer, qui a les mêmes coordonnées que le pixel à l'aplomb du centre de la fenêtre, une valeur A. Dans ce cas on dit que les érosions et les dilatations sont faites en niveau de gris. Il en résulte immédiatement que les fermetures et les ouvertures sont également réalisées en niveau de gris. Par contre si les images de base sont en binaire, ou même si l'on définit un seuil en deçà et au delà duquel on considère que le niveau de gris est 0 ou 1, on réalise corrélativement des fermetures et des ouvertures en binaire. La forme géométrique de la fenêtre d'exploration est tout à fait liée au résultat qu'on cherche à atteindre.

Si on considère qu'on veut obtenir des images de 512 X 512 pixels avec une face d'entrée de l'intensificateur d'images dont le diamètre est typiquement de l'ordre de 30 cm, on en déduit que la distance qui sépare 2 pixels voisins est de l'ordre de 0.5 mm. Si on choisit alors comme on vient de l'indiquer une fenêtre circulaire, dont le diamètre vaut 10 pixels (5mm) alors que la largeur de l'image des montants, même distordue, est de toute façon inférieure à 10 pixels, on peut avec une opération de fermeture de l'image obtenir le contour 59 (figure 6). En effet pendant l'opération de dilatation de cette fermeture on attribue A comme valeur de niveau de gris à tous les centres de fenêtre donc à tous les points de l'image dilatée. Si A varie un peu la variation lente de A est ainsi préservée. En définitive on a restauré la composante continue du fond d'image, ne comportant pas les pics 54. Pendant l'opération suivante d'érosion de cette fermeture, on attribue $\alpha$ comme niveau de gris à tous les points de l'image érodée correspondant au centre de la fenêtre dans l'image dilatée obtenue préalablement. On pourrait montrer que cette opération de fermeture est revenue à filtrer les pics 54 de manière à ne plus laisser subsister que le contour 59.

Si on appelle

CLOSE(I)

l'image dilatée puis érodée de l'image de base I, on se rend compte qu'on peut faire une soustraction, pixel à pixel, de l'image de base I de cette image dilatée-érodée. On obtient alors le contour 62 également montré sur la figure 6 dans laquelle le bruit de fond a disparu. L'image 62 est donc équivalente à la collection (figure 5) des coordonnées des pixels mis en relation avec leur niveau de grille NG stationnarisés. Dans la suite de cet exposé on appellera M cette image numérique stationnarisée.

Par une opération 63 on recherche ensuite dans l'image M la position des montants. La recherche d'un montant consiste, en ayant trouvé les coordonnées d'au moins un pixel qui appartient à coup sûr à un montant, à explorer les niveaux de gris des pixels immédiatement voisins pour tenter de déterminer s'ils appartiennent au même montant. Cette opération n'est cependant pas facilitée lorsque l'on arrive par exploration automatique à l'endroit des croisements des montants horizontaux et verticaux. En effet le processus automatique d'exploration risque de quitter un montant d'un type donné, par exemple vertical, pour se mettre alors à explorer un montant d'un autre type donné, par exemple horizontal. Pour éviter ce risque on crée deux images à partir de l'image M. Une première image ne comporte que les montants verticaux, et une deuxième image ne comporte que les montants horizontaux. Dans ce but, on fait subir à l'image M deux opérations d'ouverture. Une première opération d'ouverture

OPEN (M, 57)

permet de donner l'image de l'ouverture de l'image M (érosion suivie de dilatation) par le segment 57. On remarque que lors de la phase d'érosion correspondant à cette ouverture, tous les niveaux de gris des pixels se trouvant hors des montants horizontaux se trouvent annulés. En effet le segment 57 est horizontal, et dès que ce segment n'explore plus directement un montant horizontal, il explore en partie un montant vertical. Et au moins un des pixels à l'aplomb de cette fenêtre possède alors un niveau de gris nul ou très faible. Lors de l'opération de dilatation suivante, les pixels des montants verticaux ne sont pas recréés. De sorte que le segment 57 lors de cette opération d'ouverture, permet de produire une image des montants horizontaux seuls. Une image des montants horizontaux ou de ligne est caractérisée par la collection des adresses x, $y_j$ dont le niveau de gris associé n'est ni nul, ni très faible. On refait la même opération d'ouverture mais avec le segment 58. Cette fois on détermine une image de montants verticaux, de colonnes, c'est à dire la collection des pixels de coordonnées $x_i$, y dont le niveau de gris associé n'est pas nul.

En définitive avec ces deux opérations on obtient une image de lignes ou une image de colonnes dans laquelle il n'y a plus de pont entre les lignes ni entre les colonnes. La figure 3 montre de ce point de vue une image de colonnes dans laquelle les montants horizontaux ne sont plus représentés.

On peut dire que l'on a alors affaire dans ces images de montants verticaux ou de montants horizontaux à des ensembles disjoints. Dans une opération d'étiquetage, on attribue maintenant à chacun de ces ensembles un numéro, respectivement de colonne ou de ligne. En pratique ce numéro représente respectivement l'abscisse ou l'ordonnée du montant dans l'image virtuelle de la mire. Dans ce but on explore ces images de colonne ou de ligne de manière à attribuer un numéro a chaque colonne ou à chaque ligne. Un numéro de ligne sera un numéro $Y_1$, $Y_2$, ou plus généralement $Y_J$ et ce numéro de ligne sera attribué comme dimension supplémentaire à la collection des pixels dont les adresses x, $y_j$ ont montré qu'elles appartenaient à un même ensemble. Par exemple, pour déterminer la collection des pixels $x_i$, y appartenant au montant 8 on explore le contenu d'information des pixels appartenant à un profil en se déplaçant de la gauche vers la droite. On teste le contenu d'informations de chaque adresse d'élément d'image appartenant à ce profil.

Par exemple quand on arrive au pixel 10 on lui attribue un numéro. Ce numéro correspondra au numéro du montant n°8. Les pixels 11, 12, 9, 13 et 14 sont ensuite explorés et reçoivent le même numéro. Le pixel 64 ne reçoit pas de numéro. Son niveau de gris est trop faible : il n'appartient pas à un montant de même que les pixels suivants jusqu'à ce qu'on rencontre à nouveau sur le même profil des pixels dont le contenu d'information ressemble à celui des pixels 9 à 14. Dans ce cas on leur attribue un numéro de colonne incrémenté d'une unité. Ainsi de suite jusqu'au bord latéral droit de l'image. Ayant ainsi marqué de pixels appartenant à chaque colonne, on suit ensuite chacune d'entre elles vers le haut puis vers le bas en passant d'un pixel à son voisin de niveau de gris le plus élevé. Pendant le parcours on attribue à ses pixels $x_i,y$ son numéro $X_i$. Ainsi de suite, on crée la collection 69 des adresses des éléments d'images correspondant à la colonne $Y_i$, la collection 70 des adresses des éléments d'images appartenant à la colonne $Y_{l+1}$ et ainsi de suite.

On peut réitérer cette opération en explorant non plus des montants verticaux 15 mais des montants horizontaux. On obtient alors les collections 66, 67 et suivantes des adresses des éléments d'images appartenant à des lignes de numéro $Y_J$ ou $X_{J+1}$.

Par une opération 71 on peut rechercher les intersections grossières des montants. Ces intersections sont représentées par les collections des éléments d'images d'adresses $x_i$ et $y_j$ correspondant respectivement à des colonnes $X_l$ et $Y_J$ en même temps. On obtient ainsi les collections 72 à 73 de pixels attribués à chacune des intersections $X_l$, $Y_J$. En pratique l'étape 71 peut-être exécutée en même temps que la détermination des pixels de la deuxième famille des montants.

Les figures 7a et 7b montrent une particularité de la mire qui permet une précision de la correction de la distorsion inférieur au dixième de pixel. On pourrait réaliser la mire 6 (figure 7b) au moyen d'un arrangement de fils absorbants 74 par exemple en plomb ou en un autre métal. Mais la réalisation d'une mire avec de tels fils présente deux inconvénients. Premièrement sur les bords 75 et 76 des fils, la hauteur traversée par le rayonnement X est inférieur à la hauteur traversée au centre de ces fils. En conséquence les niveaux de gris détectés correspondant à l'aplomb de ces endroits traversés ne sont pas homogènes avec les niveaux de gris détectés à l'aplomb du centre des fils 74. En outre, deuxième inconvénient, pour pouvoir tenir mécaniquement, les fils doivent être réalisés avec un certain diamètre. De sorte que l'on atteint rapidement la saturation d'absorption. En définitive à l'aplomb de la partie centrale du fil 74 le signal de niveau de gris est un plat 77. Il n'est pas possible avec un tel plat de savoir exactement où se trouve le centre du fil 74.

Dans une réalisation préférée la mire est réalisée par photogravure d'une couche d'or d'épaisseur 78. L'épaisseur de cette couche est de préférence de 0,050mm. Cette photogravure est réalisée sur une plaque de verre Cette solution présente l'avantage que la mire est quasiment indéformable. En outre, l'or est un matériau parfaitement maîtrisé en photogravure. La maîtrise de la photogravure de l'or est de l'ordre de 1 à 5 millièmes de millimètre. De plus, l'or est également plus absorbant que le plomb et donc 0.05 mm d'épaisseur suffisent pour provoquer aux puissances de rayons X régulièrement utilisées un signal d'absorption non saturé. Enfin, la solution de la photogravure permet d'obtenir des flancs raides 79 de rubans 8 constituant les montants de la mire. De cette façon le phénomène d'évolution progressive du niveau de gris des pixels voisins n'est représentatif que de l'illumination X et non d'un phénomène parasite ajouté. La mire n'apporte pas d'imprécision propre.

La figure 7a montre selon un profil donné les niveaux de gris relevés pour des pixels voisins et correspondant à une colonne numérotée $X_i$. Dans l'invention on recherche la position du centre de gravité de l'illumination X pour chaque profil. L'abscisse $x_{gi}$ de ce barycentre est calculée sur la valeur des niveaux de gris $NG_i$ selon l'équation suivante

$$x_{gi} = \Sigma(x_i.NG_i)/\Sigma(NG_i).$$

Ceci permet de déterminer, en fraction de pixel, l'abscisse du barycentre 80. On effectue la même opération (fig 5) 81 de recherche de position du barycentre pour tous les profils appartenant à une même collection. On obtient ainsi par exemple les barycentres 82 à 86 pour la colonne $X_l$. De même on obtiendrait d'autres barycentres pour la ligne $Y_J$ ou plutôt pour un segment de cette ligne situé sur l'intersection grossière des montants.

Puis on calcule l'équation, de type $y = ax + b$, d'une droite 87 dite des moindres carrés dont la somme des distances telles que 88, élevée au carré, de cette droite aux barycentres trouvés soit la plus faible possible. Ces calculs sont de type connu et sont tels que

$$a = (N\Sigma x_j.y_j - \Sigma x_j. \Sigma y_j)/\sigma$$
$$b = (\Sigma x_j^2.\Sigma y_j - \Sigma x_j.y_j.\Sigma x_j)/\sigma$$

où N est le nombre de points de la régression, et où $\sigma$ est donné par

$$\sigma = N\Sigma x_j^2 - (\Sigma x_j)^2.$$

Une fois connue l'équation de l'image 87 du centre d'un montant, on recommence la même opération pour tous les autres segments, verticaux ou horizontaux de l'image. Ces opérations 89 ont pour résultat une collection de coefficient $(a_1, a_2, b_1, b_2)$, paramétrant des segments correspondant aux intersections des colonnes $X_l$ et $Y_J$. Dans une opération ultérieure 90 de type analytique on calcule les coordonnées des points d'intersection des segments 87 qui se coupent. Les coordonnées de ces intersections correspondent en définitive aux coordonnées 23 à 27 (figure 4) de l'image distordue de la mire. Ces coordonnées d'intersections sont également associées aux couples de coordonnées $X_l$ et $Y_J$ des intersections de la mire. Or ces intersections, compte tenu de la précision de la réalisation de la mire, sont connues par avance. Par exemple ces coordonnées sont obtenue en multipliant le numéro de la colonne par le pas (mesurée sur la mire) de la mire à l'endroit correspondant. En simplifiant on peut dire qu'il est possible de calculer les vecteurs de correction tels que 35 à 39 en retranchant les valeurs $X_l$ et $Y_J$ des valeurs calculées des coordonnées des points d'intersection des montants de la mire.

La correction des déformations géométriques des images est ensuite entreprise de préférence par interpolation bi-linéaire. Une correction bi-linéaire consiste à calculer la correction de distorsion à affecter à un pixel repéré dans une maille (à deux dimensions) en fonction des corrections de distorsion à appliquer à chacun des sommets de la maille. Les corrections des sommets sont combinées entre elles selon une pondération qui tient compte de l'écart relatif du pixel par rapport à chacun des sommets. On notera cependant que du fait que les corrections de distorsion des sommets des mailles de la mire sont données en fraction de pixel, la plupart du temps les coordonnées corrigées des pixels de l'image acquise tombent aussi entre quatre pixels. On peut faire alors subir à ce pixel une correction de gris. Cette restauration est menée par une deuxième correction bi-linéaire en prenant en compte quatre pixels corrigés qui entourent un pixel de l'image a visualiser.

## Revendications

1.  Procédé de correction de la distorsion d'images radiologiques acquises avec un tube (3) intensificateur de luminance, ces images comportant une collection (62) d'adresses de points d'image en relation avec des niveaux de gris attribués à ces points, dans lequel:
    -   on acquiert (1-6) l'image réelle d'une mire (6) placée sur la face d'entrée de ce tube,
    -   on évalue la distorsion (35-39) de cette image de la mire par rapport à son allure théorique attendue,
    -   et on corrige les images radiologiques en fonction de cette évaluation, caractérisé en ce que pour acquérir l'image réelle de la mire
    -   on réalise une mire amovible par photogravure (79) de montants en une couche métallique,
    -   on recherche automatiquement dans l'image réelle les montants de la mire, et
    -   on calcule dans l'image réelle de la mire la position du barycentre de l'illumination X d'un montant de cette mire de façon à obtenir une précision en fraction de pixel sur les coordonnées des montants de la mire.

2.  Procédé selon la revendication 1 caractérisé en ce que pour la photogravure de la couche métallique
    -   on réalise une couche métallique dont l'épaisseur est de l'ordre de 50 micromètres, et en ce que
    -   on munit la grille d'un quadrillage régulier de montants (7-8), ces montants étant formés par des rubans métalliques gravés de largeur de l'ordre d'un millimètre et espacés les uns des autres d'environ dix millimètres.

3.  Procédé de correction de la distorsion d'images radiologiques acquises avec un tube (3) intensificateur de luminance, ces images comportant une collection (62) d'adresses de points d'image en relation avec des niveaux de gris attribués à ces points, dans lequel:
    -   on acquiert (1-6) l'image réelle d'une mire (6) placée sur la face d'entrée de ce tube,
    -   on évalue la distorsion (35-39) de cette image de la mire par rapport à son allure théorique attendue,
    -   et on corrige les images radiologiques en fonction de cette évaluation, caractérisé en ce que pour acquérir l'image réelle de la mire
    -   on réalise une mire amovible par photogravure (79) d'une couche métallique
    -   pour la photogravure de la couche métallique on réalise une couche d'or dont l'épaisseur est de l'ordre de 50 micromètres, et en ce que
    -   on munit la grille d'un quadrillage régulier de montants (7-8), ces montants étant formés par des rubans métalliques gravés de largeur de l'ordre d'un millimètre et espacés les uns des autres d'environ dix millimètres.

4.  Procédé de correction de la distorsion d'images radiologiques acquises avec un tube (3) intensificateur de luminance, ces images comportant une collection (62) d'adresses de points d'image en relation avec des niveaux de gris attribués à ces points, dans lequel:
    -   on acquiert (1-6) l'image réelle d'une mire (6) formée de montants horizontaux et verticaux, cette mire étant placée sur la face d'entrée de ce tube,
    -   on évalue la distorsion (35-39) de cette image de la mire par rapport à son allure théorique attendue,
    -   et on corrige les images radiologiques en fonction de cette évaluation. caractérisé en ce que pour évaluer
    -   on détecte automatiquement la position de points de contrôle (23-27),
    -   en recherchant, par des opérations de morphologie mathématique, des positions de montants d'un même type,
    -   en suivant et en étiquetant ces montants,
    -   et en repérant et en étiquetant des points d'intersection de montants de deux types
    -   et en ce qu'on estime des déplacements localisés de ces points d'intersection.

5.  Procédé selon la revendication 4 caractérisé en ce qu'on effectue des opérations de morphologie mathématique en niveau de gris.

6. Procédé selon la revendication 4 ou la revendication 5 caractérisé en ce que pour rechercher, au préalable,

    - on stationnarise (56) le fond de l'image par une opération de morphologie mathématique

7. Procédé selon l'une quelconques des revendications 4 à 6 caractérisé en ce que pour rechercher on crée des images de montants d'un même type.

8. Procédé selon l'une quelconque des revendications 4 à 7 caractérisé en ce que pour estimer les déplacements

    - on estime la position d'un point de contrôle dans l'image par l'intersection de deux segments droits choisis appartenant chacun à un des montants à proximité de ce point.

9. Procédé selon la revendication 8 et la revendication 1 caractérisé en ce que pour choisir les segments

    - on calcule, le long de ce montant, et pour des points d'images alignés selon un profil perpendiculaire à ce montant, la position de barycentres (80-85) en niveau de gris,

    - et on approxime la position de l'ensemble des barycentres par un segment (87) dont la somme des distances (88) aux barycentres élevées au carré, est la plus faible.

10. Procédé selon l'une quelconque des revendications 1 à 9 caractérisé en ce que pour corriger

    - on calcule par interpolation bi-linéaire entre quatre points de contrôle estimés entourant un point quelconque, les déplacements de coordonnées à attribuer à ce point.

11. Procédé selon l'une quelconque des revendications 1 à 10 caractérisé en ce que pour corriger, en outre,

    - on restaure le niveau de gris d'un point d'image par interpolation bi-linéaire sur un voisinage de quatre points d'image voisins.

12. Procédé selon l'une quelconque des revendications 1 à 11 caractérisé en ce qu'on le réitère pour diverses positions du tube dans l'espace de l'écran en ayant soin de laisser la mire à la même place sur ce tube.

**Patentansprüche**

1. Verfahren zur Korrektur der Verzerrung radiologischer Bilder, die mit einer Leuchtdichte-Verstärkungsröhre (3) gewonnen werden, wobei die Bilder eine Sammlung (62) von Bildpunktadressen aufweisen, die mit diesen Punkten zugeordneten Graustufen in Beziehung stehen, mit den folgenden Schritten:

    - Erfassen (1 - 6) des reellen Bildes eines Prüfrasters (6), das auf der Eingangsfläche der Röhre plaziert ist,

    - Bewerten der Verzerrung (35 - 39) dieses Bildes des Prüfrasters bezüglich seines erwarteten theoretischen Verlaufs, und

    - Korrektur der radiologischen Bilder in Abhängigkeit von dieser Bewertung,

    dadurch gekennzeichnet, daß zur Erfassung des reellen Bildes des Prüfrasters die folgenden Schritte durchgeführt werden:

    - Herstellen eines abnehmbaren Prüfrasters durch Photoätzung (79) von Erhöhungen in einer Metallschicht,

    - automatisches Aufsuchen der Erhöhungen des Prüfrasters in dem reellen Bild, und

    - Berechnen der Position des Schwerpunkts der Röntgenbestrahlung einer Erhöhung dieses Prüfrasters in dem reellen Bild des Prüfrasters dergestalt, daß man eine Genauigkeit von einem Pixelbruchteil für die Koordinaten der Erhöhungen des Prüfrasters erhält.

2. Verfahren nach Anspruch 1, dadurch gekennzeichnet, daß für die Photoätzung der Metallschicht die folgenden Schritte durchgeführt werden:

    - Herstellung einer Metallschicht, deren Dicke etwa 50 μm ist und

    - Ausstatten des Gitters mit einem regelmäßigen quadratischen Liniennetz von Erhöhungen (7 - 8), wobei die Erhöhungen aus geätzten Metallbändern einer Breite von etwa 1 mm bestehen, die voneinander etwa 10 mm beabstandet sind.

3. Verfahren zur Korrektur der Verzerrung radiologischer Bilder, die mit einer Leuchtdichte-Verstärkungsröhre (3) gewonnen werden, wobei die Bilder eine Sammlung (62) von Bildpunktadressen aufweisen, die mit diesen Punkten zugeordneten Graustufen in Beziehung stehen, mit den folgenden Schritten:

    - Erfassen (1 - 6) des reellen Bildes eines Prüfrasters (6), das auf der Eingangsfläche der Röhre plaziert ist,

    - Bewerten der Verzerrung (35 - 39) des Bildes des Prüfmusters bezüglich seines erwarteten theoretischen Verlaufs, und

    - Korrektur der radiologischen Bilder in Abhängigkeit von dieser Bewertung,

dadurch gekennzeichnet, daß man zur Erfassung des reellen Bildes des Prüfrasters die folgenden Schritte durchführt:
- Herstellen eines abnehmbaren Prüfrasters durch Photoätzung (79) einer Metallschicht,
- Herstellen einer Goldschicht mit einer Dicke von etwa 50 µm für die Photoätzung der Metallschicht, und
- Ausstatten des Gitters mit einem regelmäßigen quadratischen Liniennetz von Erhöhungen (7 - 8), wobei die Erhöhungen aus geätzten Metallbändern mit einer Dicke von etwa 1 mm bestehen und zueinander ungefähr 10 mm beabstandet sind.

4. Verfahren zur Korrektur der Verzerrung radiologischer Bilder, die mit einer Leuchtdichte-Verstärkungsröhre (3) gewonnen werden, wobei die Bilder eine Sammlung (62) von Bildpunktadressen aufweisen, die mit diesen Punkten zugeordneten Graustufen in Beziehung stehen, mit den folgenden Schritten:
- Erfassen (1 - 6) des reellen Bildes eines Prüfrasters (6), das aus horizontalen und vertikalen Erhöhungen besteht und auf der Eingangsfläche der Röhre plaziert ist,
- Bewerten der Verzerrung (35 - 39) des Bildes des Prüfrasters bezüglich seines erwarteten theoretischen Verlaufs, und
- Korrektur der radiologischen Bilder in Abhängigkeit von dieser Bewertung, dadurch gekennzeichnet, daß man zur Bewertung
- automatisch die Position von Kontrollpunkten (23 - 27) erfaßt,
- indem man mit Hilfe mathematischer Morphologieoperationen gleichartige Erhöhungspositionen sucht,
- indem man diesen Erhöhungen folgt und sie benennt, und
- indem man Schnittpunkte zweier Arten von Erhöhungen markiert und benennt, und
- indem man lokalisierte Verschiebungen dieser Schnittpunkte abschätzt.

5. Verfahren nach Anspruch 4, dadurch gekennzeichnet, daß man mathematische Morphologieoperationen in der Graustufe durchführt.

6. Verfahren nach Anspruch 4 oder 5, dadurch gekennzeichnet, daß man zur vorab durchgeführten Suche
- den Hintergrund des Bildes mittels einer mathematischen Morphologieoperation festhält (56).

7. Verfahren nach einem der Ansprüche 4 bis 6, dadurch gekennzeichnet, daß man zur Suche Bilder gleichartiger Erhöhungen erzeugt.

8. Verfahren nach einem der Ansprüche 4 bis 7, dadurch gekennzeichnet, daß man zur Abschätzung der Verschiebungen
- die Position eines Kontrollpunkts in dem Bild durch Schnitt zweier ausgewählter geradliniger Segmente abschätzt, die jeweils einer der Erhöhungen in der Umgebung dieses Punkts angehören.

9. Verfahren nach Anspruch 8 und 1, dadurch gekennzeichnet, daß man zur Auswahl der Segmente die folgenden Schritte durchführt:
- Berechnen der Position von Graustufen-Schwerpunkten (80 - 85) entlang der Erhöhung und für Bildpunkte, die entlang einem senkrecht zur Erhöhung verlaufenden Profil ausgerichtet sind, und
- Annähern der Position der Gesamtheit der Schwerpunkte mittels eines Segments (87), bei dem die Summe der Abstände (88) zu den Schwerpunkten zum Quadrat am kleinsten ist.

10. Verfahren nach einem der Ansprüche 1 bis 9, dadurch gekennzeichnet, daß man zur Korrektur
- mittels bilinearer Interpolation zwischen vier geschätzten Kontrollpunkten, die einen beliebigen Punkt umgeben, die diesem Punkt zuzuordnenden Koordinatenverschiebungen berechnet.

11. Verfahren nach einem der Ansprüche 1 bis 10, dadurch gekennzeichnet, daß man zur Korrektur außerdem
- die Graustufe eines Bildpunkts mittels bilinearer Interpolation über einer Umgebung von vier benachbarten Bildpunkten wiederherstellt.

12. Verfahren nach einem der Ansprüche 1 bis 11, dadurch gekennzeichnet, daß man es für verschiedene Positionen der Röhre in dem Raum des Schirms wiederholt, wobei man darauf achtet, daß man das Prüfraster an der gleichen Stelle auf der Röhre läßt.

## Claims

1. Method for correcting the distortion of radiological images obtained with a luminance intensifier tube (3), these images having a collection (62) of image point addresses related to grey levels attributed to these points, in which:
- the actual image of a pattern (6) positioned on the input face of this tube is obtained (1-6),
- the distortion (35-39) of this image of the pattern is evaluated with respect to its expected theoretical appearance,
- and the radiological images are corrected in accordance with this evaluation, characterized in that, to obtain the actual image of the pattern,
- a removable pattern is produced by photoengraving (79) members in a metallic layer,
- the members in the pattern are sought automatically in the actual image, and
- the position of the barycentre of the illumination X of a member in this pattern is calculated in the actual image of the pattern so as to obtain a precision to a fraction of a pixel with regard to the coordinates of the members in the pattern.

2. Method according to Claim 1, characterized in that, for the photoengraving of the metallic layer,
- a metallic layer is produced, the thickness of which is approximately 50 micrometres, and in that
- the grid is provided with a regular chequer of members (7-8), these members being formed by engraved metal strips approximately one millimetre in width and spaced apart from each other by approximately ten millimetres.

3. Method for correcting the distortion of radiological images obtained with a luminance intensifier tube (3), these images having a collection (62) of image point addresses related to grey levels attributed to these points, in which:
- the actual image of a pattern (6) positioned on the input face of this tube is obtained (1-6),
- the distortion (35-39) of this image of the pattern is evaluated with respect to its expected theoretical appearance,
- and the radiological images are corrected in accordance with this evaluation, characterized in that, to obtain the actual image of the pattern,
- a removable pattern is produced by photoengraving (79) a metallic layer,
- for photoengraving the metallic layer, a layer of gold with a thickness of about 50 micrometres is produced, and in that
- the grid is provided with a regular chequer of members (7-8), these members being formed by engraved metal strips approximately one millimetre in width and spaced apart from each other by approximately ten millimetres.

4. Method for correcting the distortion of radiological images obtained with a luminance intensifier tube (3), these images having a collection (62) of image point addresses related to grey levels attributed to these points, in which:
- the actual image of a pattern (6) formed from horizontal and vertical members is obtained (1-6), this pattern being positioned on the input face of this tube,
- the distortion (35-39) of this image of the pattern is evaluated with respect to its expected theoretical appearance,
- and the radiological images are corrected in accordance with this evaluation, characterized in that, for the evaluation,
- the position of control points (23-27) are automatically detected,
- by seeking, through mathematical morphology operations, positions of members of the same type,
- by monitoring and labelling these members,
- and by locating and labelling intersection points of members of two types,
- and in that localized movements of these intersection points are estimated.

5. Method according to Claim 4, characterized in that grey level mathematical morphology operations are carried out.

6. Method according to Claim 4 or Claim 5, characterized in that, in order to carry out a search,
- the background of the image is first fixed (56) through a mathematical morphology operation.

7. Method according to any one of Claims 4 to 6, characterized in that, in order to carry out a search, images of members of the same type are created.

8. Method according to any one of Claims 4 to 7, characterized in that, to estimate the movements,
- the position of a control point in the image is estimated by the intersection of two selected straight segments, each one belonging to one of the members near this point.

9. Method according to Claim 8 and Claim 1, characterized in that, to select the segments,
- along this member and for image points aligned in a profile perpendicular to this member, the grey level barycentre positions (80-85) are calculated,
- and the position of all the barycentres is approximated by a segment (87) for which the sum of the squared distances (88) to the barycentres is the lowest.

10. Method according to any one of Claims 1 to 9, characterized in that, in order to effect a correction,

- through bilinear interpolation between four estimated control points surrounding any point, the movements of coordinates to be attributed to this point are calculated.

11. Method according to any one of Claims 1 to 10, characterized in that, additionally, in order to effect a correction,
- the grey level of an image point is restored through bilinear interpolation over a proximity of four adjoining image points.

12. Method according to any one of Claims 1 to 11, characterized in that it is repeated for various positions of the tube in the space of the screen, ensuring that the pattern is left in the same position on this tube.

FIG_1

FIG_2

FIG_3

FIG_4

FIG_5

55

| x | y | NG | 62 |
|---|---|----|----|

63

66

| x | $y_j$ | NG | $N°Y_j$ | 67 |
|---|-------|----|---------|----|

69

| $x_i$ | y | NG | $N°X_I$ | 70 |
|-------|---|----|---------|----|

71

72

| $x_i$ | $y_j$ | NG | $X_I Y_j$ | 73 |
|-------|-------|----|-----------|----|

81

| $x_{gi}$ | $y_{gj}$ | | $X_I Y_J$ |
|----------|----------|---|-----------|

89

| $(a_1, a_2, b_1, b_2) i \cdot j$ | $X_I Y_J$ |
|----------------------------------|-----------|

90

| $\Delta X_i$ | $\Delta Y_j$ | $X_I Y_J$ |
|--------------|--------------|-----------|

FIG_6

FIG_7-a

FIG_7-b